# EUROPEAN PATENT APPLICATION

(11) **EP 4 506 326 A1**
(43) Date of publication of application: **12.02.2025**
(21) Application number: 23780661.7
(22) Date of filing: 29.03.2023
(51) Int. Cl.: C07C 2/24, B01J 27/18, B01J 27/232, B01J 35/10, C07B 61/00, C07C 11/113

(54) **METHOD FOR PRODUCING OLEFIN DIMER, AND OLEFIN DIMERIZATION CATALYST**

(30) Priority: 30.03.2022 JP 2022056381; 30.03.2022 JP 2022056382
(71) Applicant: Mitsui Chemicals, Inc., Tokyo 104-0028 (JP)
(72) Inventor: KAWAHARA Jun, Sodegaura-shi, Chiba 299-0265 (JP); TAKAHASHI Naoya, Sodegaura-shi, Chiba 299-0265 (JP); NIISHIRO Ryo, Sodegaura-shi, Chiba 299-0265 (JP)
(74) Representative: Hoffmann Eitle
(86) International application number: PCT/JP2023/012750
(87) International publication number: WO 2023/190652

(57) **Abstract**

A method for producing an olefin dimer, including a step of subjecting an olefin containing an olefin having from 4 to 10 carbon atoms to a dimerization reaction in the presence of an olefin dimerization catalyst containing a compound (B) represented by BₙX (wherein B is Na or K, X is CO₃, SO₄, SiO₃, F, Cl, or Br, and n is an integer of 1 or 2 determined by a valence of X), and an alkali metal (D), a content ratio of which is in a specific range, and an olefin dimerization catalyst in which a pore diameter and a volume of pores of the compound (B) are within specific ranges.

## Description

### Technical Field

The present disclosure relates to a method for producing an olefin dimer and an olefin dimerization catalyst.

### Background Art

Olefin dimers (including codimers; the same applies hereinafter) represented as 4-methyl-1-pentene have been used as monomers for production of polyolefins. As catalysts for production of the corresponding dimers by an olefin dimerization reaction (including a codimerization reaction; the same applies hereinafter), many basic catalysts have been proposed in the past. In particular, catalysts obtained by supporting an alkali metal on a support mainly composed of an anhydrous potassium compound are often used.

As for these catalysts, research has continuously been made to enhance their activity and selectivity to target substances. From the fact that they have high initial activity but have an insufficient catalyst life, research has also continuously been pursued to extend the catalyst life.

Efforts have also been made to improve activity, selectivity, and catalyst life by adjusting the physical properties of an anhydrous potassium compound to be used and the physical properties of a support (for example, Patent Literatures 1 to 6).

For example, Patent Literature 7 also discloses a porous formed body used as a support for an α-olefin dimerization catalyst. Such an olefin dimerization catalyst can also produce 3-methyl-1-pentene, for example, by a codimerization reaction of ethylene and butene. A polymer having an extremely high melting point is obtained from this 3-methyl-1-pentene.

When using an olefin with a large number of carbon atoms as in the production of 3-methyl-1-pentene described above, the reaction system may be in liquid form. When producing an olefin dimer using the above catalyst for a long period of time in a liquid state reaction (hereinafter may be referred to also as "liquid phase reaction"), catalyst particles may be likely collapsed (powdered). In order to solve such problems, the present inventors have proposed an embodiment in which a metal oxide is combined for use with the aforementioned olefin dimerization catalyst (for example, Patent Literature 8).

### Citation List

### Patent Literature

[Patent Literature 1] JPS58-114737A
[Patent Literature 2] JPH3-42043A
[Patent Literature 3] JPH7-222927A
[Patent Literature 4] JP2006-326418A
[Patent Literature 5] JP2008-149275A
[Patent Literature 6] USP5081094
[Patent Literature 7] WO2015/093378
[Patent Literature 8] WO2019/189636

### Summary of Invention

### Technical Problem

The olefin dimerization catalyst described in Patent Literature 8 demonstrates performance that would makes it possible to stably produce an olefin dimer even under a harsh environment for particle powdering, such as in a liquid state reaction system for producing 3-methyl-1-pentene, however, a new component such as a metal oxide is used, as a result of which the production step for the catalyst may be complicated, the time required for production of the catalyst be prolonged, and the cost be higher. A method for producing an olefin dimer, being provided with a simpler production process and using a catalyst that is hardly powdered even in a liquid phase reaction, and an olefin dimerization catalyst, are desired, if possible.

An object to be solved by one embodiment according to the present disclosure is to provide a method for producing an olefin dimer, in which powdering of catalyst during an olefin dimerization reaction is inhibited and the catalyst with a simpler configuration is used. The other object is also to provide an olefin dimerization catalyst suitable for, for example, the method for producing an olefin dimer.

### Solution to Problem

The present inventors have found as a result of their investigations that an olefin dimerization catalyst used, an alkali metal content ratio of which is in a specific range, inhibits powdering of the catalyst during an olefin dimerization reaction and is excellent in reaction selectivity.

That is, means for solving the aforementioned problems include the following embodiments.
<1> A method for producing an olefin dimer, the method comprising a step of subjecting an olefin comprising an olefin having from 4 to 10 carbon atoms to a dimerization reaction in the presence of an olefin dimerization catalyst comprising a compound (B) represented by BₙX, wherein B is Na or K, X is CO₃, SO₄, SiO₃, F, Cl, or Br, and n is an integer of 1 or 2 determined by a valence of X, and an alkali metal (D), wherein a content ratio of the alkali metal (D) is from 3.0 to 12.5% by mass based on 100% by mass of the total amount of the compound (B) and the alkali metal (D).
<2> The method for producing an olefin dimer according to <1>, wherein a content ratio of the alkali metal (D) is from 4.0 to 12.5% by mass based on 100% by mass of the total amount of the compound (B) and the alkali metal (D).
<3> The method for producing an olefin dimer according to <1>, wherein a content ratio of the alkali metal (D) is from 4.8 to 12.5% by mass based on 100% by mass of the total amount of the compound (B) and the alkali metal (D).
<4> The method for producing an olefin dimer according to <1>, wherein a content ratio of the alkali metal (D) is from 5.2 to 10.5% by mass based on 100% by mass of the total amount of the compound (B) and the alkali metal (D).
<5> The method for producing an olefin dimer according to any one of from <1> to <4>, wherein the compound (B) has a volume of pores with a pore diameter of from 0.05 µm to 10 um of from 0.01 mL/g to 0.09 mL/g.
<6> An olefin dimerization catalyst, comprising: a compound (B) represented by BₙX, wherein B is Na or K, X is CO₃, SO₄, SiO₃, F, Cl, or Br, and n is an integer of 1 or 2 determined by a valence of X; and an alkali metal (D),
   wherein the compound (B) has a volume of pores with a pore diameter of from 0.05 µm to 10 µm of from 0.01 mL/g to 0.12 mL/g, and
   a content ratio of the alkali metal (D) is from 5.2 to 12.5% by mass based on 100% by mass of the total amount of the compound (B) and the alkali metal (D).
<7> The olefin dimerization catalyst according to <6>, wherein a content ratio of the alkali metal (D) is from 6.2 to 12.5% by mass.
<8> The olefin dimerization catalyst according to <6>, wherein a content ratio of the alkali metal (D) is from 6.2 to 10.5% by mass.
<9> The olefin dimerization catalyst according to any one of from <6> to <8>, wherein the compound (B) has a volume of pores with a pore diameter of from 0.05 µm to 10 um of from 0.01 mL/g to 0.09 mL/g.
<10> The olefin dimerization catalyst according to any one of from <6> to <8>, wherein the compound (B) has a volume of pores with a pore diameter of from 0.05 µm to 10 um of from 0.01 mL/g to 0.08 mL/g.
<11> The olefin dimerization catalyst according to <6>, comprising a compound (B) represented by BₙX, wherein B is Na or K, X is CO₃, SO₄, SiO₃, F, Cl, or Br, and n is an integer of 1 or 2 determined by a valence of X, and an alkali metal (D),
   wherein the compound (B) has a volume of pores with a pore diameter of from 0.05 µm to 10 µm of from 0.01 mL/g to 0.09 mL/g, and
   a molar ratio of the molar amount of all alkali metals [T'] to the molar amount of B in the compound (B) [AB'] ([AB']/[T'] (m.r.)) is from 0.60 to 0.89/(m. r.).

### Advantageous Effects of Invention

According to one embodiment according to the present disclosure, there are provided a method for producing an olefin dimer, in which a catalyst during an olefin dimerization reaction is inhibited from being powdered under harsh conditions such as a liquid phase reaction, and is excellent in its reaction selectivity, and the olefin dimer.

### Brief Description of Drawing

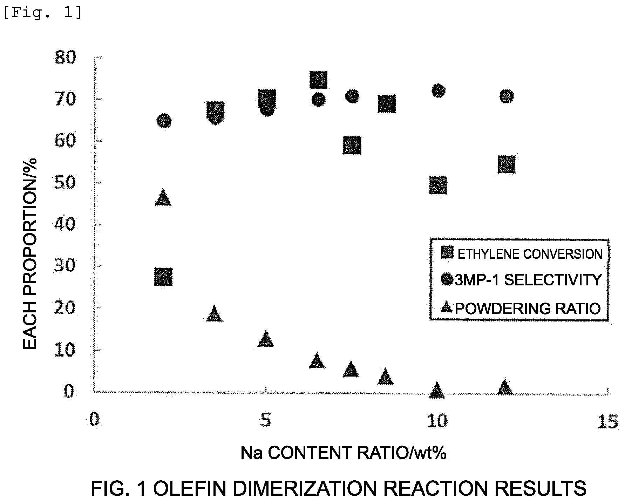

[Fig. 1] Fig. 1 shows a graph of an alkali metal content ratio of an olefin dimerization catalyst versus various reaction results in each Example and Comparative Example.

### Description of Embodiments

In the present specification, numerical ranges depicted with "from" and "to" represent ranges inclusive of numerical values that respectively appear before and after "to" as the lower limit value and the upper limit value, respectively.

In a set of numerical ranges that are stated in a stepwise manner in the present specification, the upper limit value or the lower limit value of a numerical range may be replaced with the upper limit value or the lower limit value of other numerical range. Further, in a numerical range stated in the present specification, the upper limit value or the lower limit value of the numerical range may be replaced with a value indicated in Examples.

In the present specification, a term "step" is used not only to refer to an independent step, but also to include any step that is not clearly distinguishable from other step as long as the intended purpose of the step is achieved.

### <Method for producing olefin dimer>

The method for producing an olefin dimer according to the present disclosure includes a step of subjecting an olefin having from 4 to 10 carbon atoms to a dimerization reaction in the presence of an olefin dimerization catalyst including a compound (B) represented by BₙX (wherein B is Na or K, X is CO₃, SO₄, SiO₃, F, Cl, or Br, and n is an integer of 1 or 2 determined by a valence of X), and an alkali metal (D), wherein a content ratio of the alkali metal (D) is from 3.0 to 12.5% by mass based on 100% by mass of the total amount of the compound (B) and the alkali metal (D).

The method for producing an olefin dimer uses the olefin dimerization catalyst as described above, so that the catalyst during an olefin dimerization reaction is inhibited from being powdered and is excellent in reaction selectivity.

For example, when an olefin having 4 or more carbon atoms, such as butene, is used, the reaction of the olefin is likely a liquid phase reaction. In the liquid phase reaction, catalyst particles are likely collapsed (powdered), but with the method for producing an olefin dimer of the present disclosure, the catalyst particles are unlikely to be collapsed (powdered) even in a liquid phase reaction containing an olefin having from 4 to 10 carbon atoms, thereby enabling stable production of olefin dimer for a long period of time.

Hereinafter, each component used in the method for producing an olefin dimer will be described in detail. Incidentally, details of steps included in the method for producing an olefin dimer will be described later.

### <Olefin dimerization catalyst>

The olefin dimerization catalyst that is one component of the present disclosure includes: a compound (B) represented by BₙX (wherein B is Na or K, X is CO₃, SO₄, SiO₃, F, Cl, or Br, and n is an integer of 1 or 2 determined by a valence of X); and an alkali metal (D), wherein a content ratio of the alkali metal (D) is from 3.0 to 12.5% by mass based on 100% by mass of the total amount of the compound (B) and the alkali metal (D).

Hereinafter, each requirement of the olefin dimerization catalyst will be described.

### <<Compound (B)>>

The compound (B) is represented by BₙX.

B in formula represented by BₙX is Na or K, X is CO₃, SO₄, SiO₃, F, Cl, or Br, and n is an integer of 1 or 2 determined by a valence of X.

That is, the compound (B) represented by formula BₙX is a carbonate, a sulfate, a silicate, a fluoride, a chloride or a bromide, of sodium or potassium.

The compound (B) is preferably sodium carbonate and potassium carbonate.

The compound (B) may be any one type of compound represented by BₙX, or two or more types thereof. In the case of two or more types thereof, the mixing ratio is not particularly limited.

The compound (B) may contain a carbonate of sodium or potassium, and a sulfate, a silicate, a fluoride, a chloride, or a bromide of sodium or potassium (hereinafter also referred to as a "compound (B1)").

When the compound (B) contains the compound (B1), the content ratio of compound (B1) is preferably more than 0% by mass and 30% by mass or less, more preferably more than 0% by mass and 20% by mass or less, and further preferably more than 0% by mass and 10% by mass or less, relative to the total mass of compound (B).

The compound (B) may contain at least one type of compound (B1) represented by formula of NaₙY or KₙY (wherein Y in formula is SO₄, SiO₃, F, Cl, or Br, and n is the integer of 1 or 2 determined by a valence of Y).

That is, the compound (B1) is a sulfate, a silicate, a fluoride, a chloride or a bromide of sodium or potassium.

The compound (B1) may be any one of the compounds represented by formula of NaₙY or KₙY, or a mixture of two or more thereof. When the compound (B1) is a mixture of two or more thereof, a mixing ratio of the mixture is not particularly limited.

When the compound (B) or its composition contains the compound (B1), the content ratio of the compound (B1) is preferably more than 0% by mass and 30% by mass or less, more preferably more than 0% by mass and 20% by mass or less, and further preferably more than 0% by mass and 10% by mass or less.

A shape of the compound (B) is preferably particulate in order to use it as an olefin dimerization catalyst. When the compound (B) is particulate, its particle size is, preferably from 5 um to 600 um, more preferably from 20 um to 500 um, further preferably from 50 um to 450 um, and particularly preferably from 50 um to 300 um in terms of as a median diameter (d50) at a volume static.

Even when other optional component is included, the same range is preferred.

Powder with a median diameter of from 5 µm to 600 µm has favorable flowability, making it possible to stably produce a formed body.

In the compound (B), the content ratio of particles with a particle size of 40 µm or smaller is preferably from 3% by mass to 30% by mass relative to the total mass of compound (B) .

Generally, small-size particles are known to be desirably excluded because of their bad movement during tablet compressing, however, the content ratio of particles with a particle size of 40 um or less is preferably within the above range. When the content ratio of particles with a particle size of 40 um or less is within the above range, the flowability of the raw material can be ensured, and the blocking is less likely occurred upon forming of the raw material, so that homogeneous filling of the raw material can be achieved.

The compound (B) may be a formed body. A method for forming the compound (B) is not particularly limited, and employs methods such as extrusion forming, compression forming, and granulation forming.

Among these, examples of the methods for forming the compound (B) include preferably extrusion forming and compression forming, as well as tablet compression. Incidentally, water is preferably removed from the compound (B) and a composition containing the compound (B) as much as possible.

Among the aforementioned forming methods, in view of the nature of the raw material, it is preferably formed by compression forming and particularly preferably tablet compression.

When forming the raw material by compression forming, it is typically filled in a mortar which serves as a die, and compressed with a pounder followed by forming.

When the raw material is formed by tablet compression, since the raw material is uniformly filled into a mortar, a formed body with a small variation in density tends to be obtained.

When a raw material is formed by extrusion forming, a forming raw material with enough viscosity to maintain its shape given by addition of a liquid to the raw material is passed into a die to be formed.

When an olefin dimerization catalyst contains the hydrogen carbonate compound (A) described below, the hydrogen carbonate compound (A) is dissolved in water, and therefore, when a raw material is formed by extrusion forming, a solvent in which the hydrogen carbonate compound (A) would not be dissolved (for example, organic solvent) is preferably used.

When the raw material is formed by tablet compression, the density of the tablet compressed formed body is preferably from 1.6 g/mL to 2.3 g/mL and more preferably from 1.8 g/mL to 2.2 g/mL.

The density of the tablet compressed formed body can be adjusted by controlling the compression strength.

In the case of the compound (B) being a formed body, its size and shape are not particularly limited.

The shape of the formed body can be selected depending on the conditions of, for example, a forming device, and can take any of a noodle shape, columnar shape, convex shape, ring shape, or spherical shape.

When a raw material is formed by tablet compression, the shape of a formed body is preferably a columnar shape, convex shape, or ring shape, and a columnar shape is more preferred from the viewpoints of easiness to form and strength.

Commercial tablet compressing machines can be used, and may be either a rotary type or a press type and a device with an optimum scale can appropriately be selected depending on the amount of production. When forming a raw material into a columnar shape using a tablet compressing machine, the raw material is formed into a formed body with a diameter of from 2 mm to 5 mm and a height of from 2 mm to 6 mm.

Within the above range of the size of the formed body, the formed body is inhibited from being excessively small, thereby enabling inhibition of increase in the number of times of tableting. This leads to favorable productivity and reduction in costs.

Within the above range of the size of the formed body, when the formed body is heat-treated and then applied as a support of, for example, an olefin dimerization catalyst, a raw material and product are likely to diffuse favorably within the reaction system, so that the reaction activity and reaction selectivity of an olefin dimerization reaction is likely improved.

### <<Pore volume>>

The compound (B) preferably has a volume of pores with a pore diameter of from 0.05 um to 10 um, of from 0.01 mL/g to 0.12 mL/g.

The lower limit value of the volume of pores is preferably 0.02 mL/g and more preferably 0.025 mL/g. The upper limit value of the volume of pores is, on the other hand, preferably 0.10 mL/g, more preferably 0.09 mL/g, further preferably 0.085 mL/g, and particularly preferably 0.080 mL/g. Within the above range of the volume of pores, the olefin dimerization reaction described below tends to proceed stably for a long period of time.

The compound (B) preferably has a median pore diameter of pores with a pore diameter of from 0.001 µm to 10 um of more than 0.05 µm and 0.35 µm or less. For example, in a case in which the present catalyst of the present application is filled in a fixed bed and used, the above requirement that was met is preferred from the viewpoint of for example, powdering inhibition of catalyst particles (one requirement for prolonging a catalyst life) and handling upon filling and discharge of the catalyst.

The pore diameter can be adjusted by, for example, a proportion of the hydrogen carbonate compound (A) which will be described later and calcination conditions.

The above requirement of catalyst shape is also the same for the olefin dimerization catalyst containing an olefin having from 4 to 10 carbon atoms, which will be described later. A method for producing an olefin dimerization catalyst also depends on manners and conditions of the production method, but often strongly depends on the shape of the compound (B).

### <Alkali metal (D)>

The alkali metal (D) is preferably a metal selected from the group consisting of sodium and potassium and more preferably sodium.

Herein, the alkali metal (D) is a metal having a valence of 0, which is not ionized. The alkali metal (D) may contain a component in addition to the alkali metal when the purity of the alkali metal is 90% or more. Examples of the components other than the alkali metal include, for example, various oxides or hydroxides of elements other than elements in Group 1 of the Periodic Table such as lithium and potassium, and a metal element other than elements in Group 1 of the Periodic Table.

The content ratio of alkali metal (D) (may be referred to as h) in the olefin dimerization catalyst of the present disclosure is from 3.0% by mass to 12.5% by mass, based on 100% by mass of the total amount of alkali metal (D) and compound (B).

The lower limit value of the content ratio of alkali metal (D) is preferably 4.0% by mass, more preferably 4.8% by mass, further preferably 5.2% by mass, further preferably 5.5% by mass, further preferably 5.9% by mass, particularly preferably 6.2% by mass, especially preferably 6.3% by mass, and most preferably 6.4% by mass. The upper limit value of the content ratio of alkali metal (D) is, on the other hand, preferably 11.0% by mass and more preferably 10.5% by mass. It is further preferably 9.5% by mass, particularly preferably 9.0% by mass, and most preferably 8.5% by mass.

Not only in a case in which an olefin having from 4 to 10 carbon atoms is contained, but also in other embodiments, the olefin dimerization catalyst of the present disclosure may function as a suitable dimerization catalyst from the viewpoint of for example, reaction durability. The content ratio of alkali metal (D) in such a case, is from 5.2% by mass to 12.5% by mass based on 100% by mass of the total amount of alkali metal (D) and compound (B). The lower limit value of the content ratio of alkali metal (D) is preferably 5.5% by mass, more preferably 5.9% by mass, further preferably 6.2% by mass, particularly preferably 6.3% by mass, and most preferably 6.4% by mass. The upper limit value of the preferred content ratio of alkali metal (D) is, on the other hand, preferably 11.0% by mass and more preferably 10.5% by mass. It is more preferably 9.5% by mass, particularly preferably 9.0% by mass, and most preferably 9.0% by mass.

The olefin dimerization catalyst of the present disclosure may contain other components in addition to the compound (B) and the alkali metal (D) as long as the olefin dimerization catalyst is within the scope of the purpose of the present disclosure.

Examples of other components include, for example, an alkali metal hydrogen carbonate compound (A) and graphite (C) .

### -Hydrogen carbonate compound (A)-

Examples of the alkali metal hydrogen carbonate compound (A) (hereinafter also simply referred to as "hydrogen carbonate compound (A)") suitably include the compound represented by formula: AHCO₃ (wherein in formula, A represents an alkali metal). A in formula is preferably Na or K.

Examples of the hydrogen carbonate compound (A) include at least one of sodium hydrogen carbonate (NaHCOs) and potassium hydrogen carbonate (KHCO₃). When the hydrogen carbonate compound (A) contains both sodium hydrogen carbonate (NaHCO₃) and potassium hydrogen carbonate (KHCO₃), their mixing ratio is not particularly limited.

When the olefin dimerization catalyst contains the hydrogen carbonate compound (A), a preferred proportion of the hydrogen carbonate compound (A) is preferably 20 parts by mass or less, more preferably 15 parts by mass or less, further preferably 10 parts by mass or less, and particularly preferably 5 parts by mass or less, relative to 100 parts by mass of the total of the hydrogen carbonate compound (A) and the compound (B).

The hydrogen carbonate compound (A) is more preferably potassium hydrogen carbonate (KHCO₃) in which A in the above formula is K, for example, from the viewpoint of adjusting a pore size of the olefin dimerization catalyst of the present disclosure to a larger size.

### -Graphite (C)-

The olefin dimerization catalyst may further contain a graphite (C).

When the olefin dimerization catalyst further contains the graphite (C) and the raw material containing the graphite (C) is formed, for example, by the compression forming (for example, tablet compression) described below, the movement of a mortar and a pounder is likely smooth, so that the density of the resulting formed body is likely stable.

The nature of the graphite (C) is not particularly limited, and any graphite that is commonly used as a lubricant upon forming may be used.

The graphite (C) that is commonly used, has a median diameter (d50) of from 5 µm to 500 µm in terms of a volume statistic and examples of the graphite (C) include a graphite having the specific surface area measured by a BET method, of from 0.1 m²/g to 300 m²/g.

The graphite (C) may be a natural graphite or an artificial graphite.

The content of graphite (C) in the olefin dimerization catalyst can be arbitrarily set as long as it can be formed by for example, compression forming or granulation forming.

The content ratio of graphite (C) is preferably from 0.3 parts by mass to 10 parts by mass and more preferably from 0.5 parts by mass to 5 parts by mass, relative to 100 parts by mass of the hydrogen carbonate compound (A), which is an optional component, and the compound (B), which is an essential ingredient.

The amount of graphite (C) added being 10 parts by mass or less tends to hardly decrease the strength of the olefin dimerization catalyst of the present disclosure. As describe above, for example, in a case in which a raw material is formed by tablet compression, the graphite (C) with the amount added of 0.3 parts by mass or more can inhibit the friction between the mortar and the pounder from being excessively large. This facilitates favorable operation of device and stabilization of the density of a formed body.

Therefore, within the above range of the amount of graphite (C) added, a formed body is likely formed. Thereby, the strength of a porous formed body obtained by heat-treating the formed body is likely ensured.

The olefin dimerization catalyst may further contain a component in addition to the hydrogen carbonate compound (A), graphite (C), and alkali metal (D).

Examples of such other compounds include ammonium hydrogen carbonate. When a raw material contains ammonium hydrogen carbonate, the volume of pores of a porous formed body can be made larger.

The alkali metal atom in the olefin dimerization catalyst of the present disclosure can contain an atom derived from the alkali metal (D) and atoms derived from alkali metal salts of the compound (B) and the component (A), which is an optional component (corresponding to B and A in the above formula).

Examples of more preferred embodiments of the olefin dimerization catalyst of the present disclosure can include
an olefin dimerization catalyst containing the compound (B) represented by BₙX (wherein in formula, B is Na or K, X is CO₃, SO₄, SiO₃, F, Cl, or Br, and n is an integer of 1 or 2 determined by a valence of X), and an alkali metal (D),
wherein the compound (B) has a volume of pores with a pore diameter of from 0.05 um to 10 um of from 0.01 mL/g to 0.09 mL/g, and
a molar ratio of the molar amount of all alkali metals [T'] to the molar amount of B in the compound (B) [AB'] ([AB']/[T'] (m.r.)) is from 0.60 to 0.89/(m. r.). The above (m. r.) refers to a molar ratio.

However, [AB'] is an index that includes not only the amount of B in the compound (B), but also the amount of Na and K contained in a salt such as A in the compound (A), which is an optional component.

The lower limit value described above is more preferably 0.63, more preferably 0.65, and particularly preferably 0.67. The upper limit value is, on the other hand, preferably 0.88, further preferably 0.87, and particularly preferably 0.86.

Within the above numerical ranges, difficulty of particle collapse and olefin dimerization reaction performance are well balanced. The reason for this assumption will be described later.

The above parameter can be calculated from the elemental analysis value of the alkali metal of the olefin dimerization catalyst of the present disclosure and the elemental analysis value of the alkali metal after having removed the alkali metal (D) from the olefin dimerization catalyst. Of course, the above parameter can also be calculated from a feed ratio of each component upon preparation of the olefin dimerization catalyst.

As described above, the olefin dimerization catalyst of the present disclosure is in the form containing the alkali metal (D), but this alkali metal (D) is considered to be present in the form of the alkali metal itself, not as a compound such as a salt. As is well known, the alkali metal is relatively easy to melt and vaporize, and tends to have a lower melting point and boiling point than a salt compound of the alkali metal. For this reason, setting temperature of the olefin dimerization catalyst to the melting point of the alkali metal (D) or higher and the melting point of a salt compound such as the compound (B) or lower makes it possible to remove the alkali metal (D) from the olefin dimerization catalyst. In addition thereto, a separation method that utilizes a boiling point difference of each component can of course be adopted as well.

As the elemental analysis method, known methods such as an ICP analysis method (high frequency inductively coupled plasma emission spectrometry) can be utilized without limitation.

### <<Method for producing olefin dimerization catalyst>>

The olefin dimerization catalyst can be obtained, for example, by contacting the above compound (B) with the alkali metal (D). This may also be considered as a method for supporting the alkali metal (D) on a support called the compound (B).

As a method for contacting the compound (B) with the alkali metal (D) to obtain an olefin dimerization catalyst, any known method can be used without limitation. Specific conditions in a contact support method as an example will be described below.

A temperature upon supporting treatment is usually in a range of from 150°C to 400°C. From the viewpoint of obtaining a catalyst with excellent catalyst activity, catalyst life, and selectivity to olefin dimerization products, the temperature upon supporting treatment is preferably in the range of from 200°C to 350°C and preferably in the range of from 200°C to 300°C. An atmosphere upon supporting treatment may be a reducing atmosphere or an inert atmosphere as long as it is not a moisture and oxidizing atmosphere. Considering safety and economic efficiency, it is preferable to carry out the supporting treatment in a nitrogen atmosphere.

Upon the supporting treatment, in order to uniformly support the alkali metal (D), a support made of a porous formed body is preferably vibrated, rotated, or stirred. The supported alkali metal (D) is known to cause an exchange reaction with an alkali metal in a support when the alkali metal (D) contacts the support under heating.

The method for producing an olefin dimerization catalyst may preferably include a step of heat-treating the compound (B) at a temperature in a range of from 100°C to 500°C.

The heat treatment time (namely, heat treatment time) is preferably 0.3 hours or longer. For example, when the hydrogen carbonate compound (A) is combined for use, it is preferable to carry out the treatment described above. The atmosphere in this case is not limited to air or inert gas. For the purpose of adjusting porosity and a volume of pores, the atmosphere may be an atmosphere containing other gas such as water vapor. When water vapor is combined for use, the amount is preferably from 1.0 g/m³ to 750,000 g/m³ and more preferably from 10.0 g/m³ to 750,000 g/m³.

The content ratio of alkali metal (D) in the olefin dimerization catalyst (hereinafter may be referred to as "support ratio") is from 3.0% by mass to 12.5% by mass based on 100% by mass of the total amount of alkali metal (D) and compound (B).

The lower limit value of the content ratio of the alkali metal (D) is preferably 4.0% by mass, more preferably 4.8% by mass, further preferably 5.2% by mass, particularly preferably 6.2% by mass, and most preferably 6.4% by mass. The upper limit value of the content ratio of the alkali metal (D) is, on the other hand, preferably 11.0% by mass and more preferably 10.5% by mass. It is further preferably 9.5% by weight, particularly preferably 9.0% by weight, and most preferably 8.5% by weight.

It can be easily presumed that an increase in the content ratio of the alkali metal (D) tends to allow catalytic activity to be enhanced, however, when the content ratio reaches a specific region, the improvement effect may be lowered. The olefin dimerization catalyst used in the method for producing an olefin dimer according to the present disclosure exhibits a relatively high level of balance between reactivity and selectivity when the content ratio of the alkali metal (D) is in a range of from 3.0% by mass to 12.5% by mass, and the performance balance tends to be hardly influenced by the alkali metal (D) content ratio. Surprisingly, the catalyst unexpectedly has excellent characteristics that an increase in the content ratio of the alkali metal makes it difficult for the catalyst to be powdered even when used under the liquid phase reaction conditions that are disadvantageous for maintaining the catalyst shape. The present inventors think that this is because the alkali metal (D) partially or totally has a function as a binder in addition to serving as a catalyst active site, which thereby may inhibit powdering of catalyst particles.

In order to achieve such a high alkali metal (D) content ratio, the olefin dimerization catalyst of the present disclosure desirably has a pore volume value as high as possible. On the other hand, the higher the pore volume value, the more likely the catalyst tends to be collapsed. The range of the pore volume value that the olefin dimerization catalyst of the present disclosure has, was selected also in consideration of the aforementioned viewpoints.

As described above, the present disclosure enables production of olefin dimer using a catalyst having a simple configuration as an olefin dimerization catalyst, exhibiting an unexpected effect that is called powdering inhibition, and excellent in selectivity and reactivity. Therefore, it is possible to stably and efficiently produce an olefin dimer for a long period of time.

The olefin dimerization catalyst of the present disclosure preferably has a volume of pores with a pore diameter of from 0.05 um to 10 um of from 0.01 mL/g to 0.12 mL/g. The lower limit value of the volume of pores of the olefin dimerization catalyst is preferably 0.02 mL/g and more preferably 0.025 mL/g. The upper limit value of the volume of pores of the olefin dimerization catalyst is, on the other hand, preferably 0.10 mL/g, more preferably 0.09 mL/g, further preferably 0.085 mL/g, particularly preferably 0.08 mL/g, and especially preferably 0.07 mL/g.

Within such ranges of the volume of pores of the olefin dimerization catalyst, the olefin dimerization reaction including an olefin having from 4 to 10 carbon atoms, which will be described later, tends to allow this reaction to proceed stably for a long period of time.

### <Dimerization reaction step>

The method for producing an olefin dimer according to the present disclosure includes a step (dimerization reaction step) of dimerizing an olefin containing an olefin having from 4 to 10 carbon atoms in the presence of an olefin dimerization catalyst. The olefin dimerization catalyst used in the dimerization reaction step is as described above.

In the dimerization reaction step, a reaction temperature in the olefin dimerization reaction is usually from 0°C to 300°C and preferably from 50°C to 200°C.

Reaction pressure is also usually normal pressure or 19.6 MPa (200 kg/cm²-G) and preferably in a range of from 1.96 MPa to 14.7 MPa (from 20 kg/cm²-G to 150 kg/cm²-G).

A state of an olefin in the olefin dimerization reaction step is preferably in a liquid phase state. The liquid phase may be in a critical state as long as it is liquid.

The olefin dimerization reaction can be carried out in a fixed bed manner or in a fluidized bed manner. Among these, a fixed bed manner is preferred.

When the dimerization reaction is carried out in a fixed bed manner, the liquid hourly space velocity (LHSV) of the olefin is usually in a range of from 0.1 hr⁻¹ to 10 hr⁻¹ and preferably in the range of from 0.5 hr⁻¹ to 5 hr⁻¹.

The unreacted olefin and product are separated from the mixture after completion of dimerization reaction according to a conventional method, and the unreacted olefin can also be recycled and reused in a reaction.

### «Olefin containing olefin having from 4 to 10 carbon atoms»

The olefin containing an olefin having from 4 to 10 carbon atoms may contain only an olefin having from 4 to 10 carbon atoms, or may contain an olefin having from 4 to 10 carbon atoms and an olefin having a carbon atom other than from 4 to 10 carbon atoms.

Specific examples of the olefin having from 4 to 10 carbon atoms include olefins such as 1-butene, cis-2-butene, trans-2-butene, isobutylene, 1-pentene, 1-hexene, 1-octene, and 1-decene.

As the olefin having a carbon atom other than from 4 to 10 carbon atoms, ethylene having 2 carbon atoms and propylene having 3 carbon atoms are preferred. A method of producing an olefin codimer by codimerizing such an olefin with an olefin having from 4 to 10 carbon atoms described above is an example of the preferred embodiment of the present disclosure.

The particularly preferred embodiment of the method for producing an olefin dimer of the present disclosure is a codimerization reaction of 1-butene or 2-butenes with ethylene or propylene, and in particular examples thereof include a method for producing 3-methyl-1-pentene by a codimerization reaction of 1-butene or 2-butenes with ethylene.

As the above ethylene and olefin having from 3 to 10 carbon atoms used in the present disclosure, not only an olefin derived from a fossil resource such as petroleum, but also a so-called bio-derived olefin such as an olefin derived from plants.

The method for producing an olefin dimer may include a step in addition to the above steps as long as the effects of the present disclosure are achieved.

Examples of other steps include, for example, a step of preparing an olefin dimerization catalyst.

### Examples

The present disclosure will be specifically described with reference to Examples, but the present disclosure is in no way limited to these Examples.

### [Measurement of pore volume and median pore diameter]

For K₂CO₃ powders used in Examples from 1 to 7 and Comparative Example 1, volumes of pores with a pore diameter (i.e., pore size) of from 0.05 um to 10 µm were each determined by mercury porosimetry using a porosimeter (model number: Auto Pore IV, manufactured by Micrometrics, Inc.). The results are shown in Table 1.

### [Calculation method of powdering ratio]

After completion of reaction for 120 hours in Examples and Comparative Examples, the total mass of a catalyst taken out from a reactor was measured. Thereafter, the prepared catalyst was separated using a sieve with a mesh opening of 500 um, and the powdering ratio (% by mass) of the catalyst was determined using the following calculation formula. It can be said that the smaller the value of the powdering ratio, the more inhibited powdering of a catalyst during an olefin dimerization reaction. Powdering ratio (% by mass) = mass of powder that passed through a sieve/total mass of the catalyst × 100

### [Ethylene conversion and 3-methyl-1-pentene selectivity]

They were determined by an ordinary method using a GC 2014 type gas chromatograph manufactured by Shimadzu Corporation. A column manufactured by Agilent Technologies Inc. (product name DB-5 (inner diameter: 0.25 mm, length: 60 m, membrane pressure: 1.0 um, and Catalog No. 122-5063)) was used.

### [Measurement of crushing strength of porous formed body]

Using a digital hardness meter (manufactured by FUJIWARA SCIENTIFIC CO., LTD., Model No. KHT-40N), the crushing strength of a porous formed body in its radial direction (i.e., barrel direction of the columnar formed body) was measured according to the method described in JIS Z8841 (1993) "Granules and agglomerates - Test method for strength".

The principle of measurement of the crushing strength involves placing a columnar porous formed body to be measured on a sample table at rest, descending a movable pressuring surface from the upper part at a constant rate, and pushing the surface against the columnar porous formed body to measure the strength when the surface crushed the formed body.

### [Example 1] (reference example for Claim 6)

### (1) Preparation of catalyst

One hundred (100) parts by mass of K₂CO₃ powder (manufactured by AGC Inc., purity: 99%) and 1.2 parts by mass of graphite (manufactured by Nippon Graphite Industries Co., Ltd., purity: 98%) were mixed uniformly, tableted as a raw material, and formed into a columnar shape with a diameter of 3.2 mm and a height of 3.2 mm to prepare a formed body. The volume of pores of the formed body was 0.18 mL/g.

Ninety six point five (96.5) parts by mass of the above formed body was dried at 300°C in a nitrogen stream for 2 hours, and then added with 3.5 parts by mass of sodium under a nitrogen stream atmosphere, and the mixture was stirred at 240°C for 4.0 hours to obtain an olefin dimerization catalyst.

Since no sodium added adhered to a support container was found, it was determined that the entire amount of sodium was supported on the formed body. The supporting ratio in this case was 5.0% by mass. The strength in the barrel direction after catalyst preparation was 2.4 kgf.

### (2) Dimerization reaction

A single tube reactor with a diameter of 18 mm was filled with 2.5 g of the catalyst prepared in (1) above, and under the conditions of an internal temperature of the reactor of 85°C and reaction pressure of 9.3 MPa, a mixed liquid of 10 wt% ethylene (diluted with 1-butene) was continuously supplied to a catalyst layer at a flow rate of 7.2 g/h to carry out a synthesis reaction of 3MP1 (3-methyl-1-pentene) by a codimerization reaction of ethylene and 1-butene. Incidentally, under the above temperature and pressure conditions, the reaction system was in a liquid phase.

The ethylene conversion determined by the above method when the flow reaction was carried out for 120 hours, was 67.7%, the 3MP1 selectivity was 65.9%, and the powdering ratio was 18.9%.

### [Example 2] (reference example for Claim 6)

(1) A catalyst was prepared and a dimerization reaction was carried out in the same manner as in Example 1 except that the amount of formed body was 95.0 parts by mass and the amount of sodium was 5.0 parts by mass in the preparation of the catalyst. The results of the ethylene conversion, 3MP1 selectivity, and powdering ratio are shown in Table 1. The strength of the catalyst was 1.4 kgf.

### [Example 3]

(1) A catalyst was prepared and a dimerization reaction was carried out in the same manner as in Example 1 except that the amount of formed body was 93.5 parts by mass and the amount of sodium was 6.5 parts by mass in the preparation of the catalyst. The strength of the catalyst was 2.0 kgf. The results are shown in Table 1.

### [Example 4]

(1) A catalyst was prepared and a dimerization reaction was carried out in the same manner as in Example 1 except that the amount of formed body was 92.5 parts by mass and the amount of sodium was 7.5 parts by mass in the preparation of the catalyst. The strength of the catalyst was 1.4 kgf. The results are shown in Table 1.

### [Example 5]

(1) A catalyst was prepared and a dimerization reaction was carried out in the same manner as in Example 1 except that the amount of formed body was 91.5 parts by mass and the amount of sodium was 8.5 parts by mass in the preparation of the catalyst. The strength of the catalyst was 1.0 kgf. The results are shown in Table 1.

### [Example 6]

(1) A catalyst was prepared and a dimerization reaction was carried out in the same manner as in Example 1 except that the amount of formed body was 90.0 parts by mass and the amount of sodium was 10.0 parts by mass in the preparation of the catalyst. The strength of the catalyst was 1.2 kgf. The results are shown in Table 1.

### [Example 7]

(1) A catalyst was prepared and a dimerization reaction was carried out in the same manner as in Example 1 except that the amount of formed body was 88.0 parts by mass and the amount of sodium was 12.0 parts by mass in the preparation of the catalyst. The strength of the catalyst was 1.0 kgf. The results are shown in Table 1.

### [Comparative Example 1]

(1) A catalyst was prepared and a dimerization reaction was carried out in the same manner as in Example 1 except that the amount of formed body was 98.0 parts by mass and the amount of sodium was 2.0 parts by mass in the preparation of the catalyst. The strength of the catalyst was 2.4 kgf. The results are shown in Table 1.

**[Table 1]**

| | Example 1 | Example 2 | Example 3 | Example 4 | Example 5 | Example 6 | Example 7 | Comparative Example 1 |
|---|---|---|---|---|---|---|---|---|
| Na amount/wt% | 3.5 | 5.0 | 6.5 | 7.5 | 8.5 | 10 | 12 | 2.0 |
| Volume of pores/(ml/g) | 0.05 | 0.05 | 0.05 | 0.05 | 0.05 | 0.05 | 0.05 | 0.05 |
| Ethylene conversion/mol% | 67.7 | 70.6 | 75.1 | 59.4 | 69.3 | 49.9 | 55.0 | 27.6 |
| 3MPI selectivity/mol% | 65.9 | 67.9 | 70.4 | 71.1 | 69.4 | 72.6 | 71.3 | 65.1 |
| Powdering ratio/wt% | 18.9 | 12.9 | 7.9 | 5.9 | 4.0 | 1.0 | 1.7 | 46.6 |
| [AB']/[T']/(m.r.)²⁾ | 0.900 | 0.861 | 0.824 | 0.801 | 0.778 | 0.745 | 0.705 | 0.941 |

| | | | | | | | | |
|---|---|---|---|---|---|---|---|---|
| 1) 3MP1 amount produced/converted ethylene (/mol%) 2) Molar ratio of the molar amount of all alkali metals [T'] to the molar amount of B in a (B) component [AB'] | | | | | | | | |

Compared to the method for producing the olefin dimer in Comparative Example 1, the method for producing the olefin dimer in Examples 1 to 7 according to the present disclosure finds that powdering of the catalyst during the olefin dimerization reaction is inhibited and the catalyst is excellent in its reaction selectivity. It is found that the catalysts of Examples 3 to 7 according to the present disclosure are also unlikely to be powdered and have excellent selectivity in the 3MP-1 synthesis reaction.

The catalyst used in the method for producing an olefin dimer according to the present disclosure also has the sufficient strength. The particle strength and powdering ratio are not, on the other hand, necessarily relevant indices.

## Claims

1. A method for producing an olefin dimer, the method comprising a step of subjecting an olefin comprising an olefin having from 4 to 10 carbon atoms to a dimerization reaction in the presence of an olefin dimerization catalyst comprising a compound (B) represented by BₙX, wherein B is Na or K, X is CO₃, SO₄, SiO₃, F, Cl, or Br, and n is an integer of 1 or 2 determined by a valence of X, and an alkali metal (D), wherein a content ratio of the alkali metal (D) is from 3.0 to 12.5% by mass based on 100% by mass of the total amount of the compound (B) and the alkali metal (D).

2. The method for producing an olefin dimer according to claim 1, wherein the content ratio of the alkali metal (D) is from 4.0 to 12.5% by mass based on 100% by mass of the total amount of the compound (B) and the alkali metal (D).

3. The method for producing an olefin dimer according to claim 1, wherein the content ratio of the alkali metal (D) is from 4.8 to 12.5% by mass based on 100% by mass of the total amount of the compound (B) and the alkali metal (D).

4. The method for producing an olefin dimer according to claim 1, wherein the content ratio of the alkali metal (D) is from 5.2 to 10.5% by mass based on 100% by mass of the total amount of the compound (B) and the alkali metal (D).

5. The method for producing an olefin dimer according to claim 1, wherein the compound (B) has a volume of pores with a pore diameter of from 0.05 µm to 10 µm of from 0.01 mL/g to 0.09 mL/g.

6. An olefin dimerization catalyst, comprising: a compound (B) represented by BₙX, wherein B is Na or K, X is CO₃, SO₄, SiO₃, F, Cl, or Br, and n is an integer of 1 or 2 determined by a valence of X; and an alkali metal (D),
wherein the compound (B) has a volume of pores with a pore diameter of from 0.05 um to 10 um of from 0.01 mL/g to 0.12 mL/g, and
a content ratio of the alkali metal (D) is from 5.2 to 12.5% by mass based on 100% by mass of the total amount of the compound (B) and the alkali metal (D).

7. The olefin dimerization catalyst according to claim 6, wherein the content ratio of the alkali metal (D) is from 6.2 to 12.5% by mass.

8. The olefin dimerization catalyst according to claim 6, wherein the content ratio of the alkali metal (D) is from 6.2 to 10.5% by mass.

9. The olefin dimerization catalyst according to claim 6, wherein the compound (B) has a volume of pores with a pore diameter of from 0.05 µm to 10 µm of from 0.01 mL/g to 0.09 mL/g.

10. The olefin dimerization catalyst according to claim 6, wherein the compound (B) has a volume of pores with a pore diameter of from 0.05 µm to 10 µm of from 0.01 mL/g to 0.08 mL/g.

11. The olefin dimerization catalyst according to claim 6, comprising a compound (B) represented by BₙX, wherein B is Na or K, X is CO₃, SO₄, SiO₃, F, Cl, or Br, and n is an integer of 1 or 2 determined by a valence of X, and an alkali metal (D),
wherein the compound (B) has a volume of pores with a pore diameter of from 0.05 um to 10 um of from 0.01 mL/g to 0.09 mL/g, and
a molar ratio of the molar amount of all alkali metals [T'] to the molar amount of B in the compound (B) [AB'] ([AB']/[T'] (m.r.)) is from 0.60 to 0.89/(m. r.).

## Amended claims

### Amended claims in accordance with Rule 137(2) EPC.

1. (Amended) A method for producing an olefin dimer, the method comprising a step of subjecting an olefin comprising an olefin having from 4 to 10 carbon atoms to a dimerization reaction in the presence of an olefin dimerization catalyst comprising a compound (B) represented by BₙX, wherein B is Na or K, X is CO₃, SO₄, SiO₃, F, Cl, or Br, and n is an integer of 1 or 2 determined by a valence of X, and an alkali metal (D), wherein the compound (B) has a volume of pores with a pore diameter of from 0.05 µm to 10 µm of from 0.01 mL/g to 0.09 mL/g, and a content ratio of the alkali metal (D) is from 3.0 to 12.5% by mass based on 100% by mass of the total amount of the compound (B) and the alkali metal (D).

2. The method for producing an olefin dimer according to claim 1, wherein the content ratio of the alkali metal (D) is from 4.0 to 12.5% by mass based on 100% by mass of the total amount of the compound (B) and the alkali metal (D).

3. The method for producing an olefin dimer according to claim 1, wherein the content ratio of the alkali metal (D) is from 4.8 to 12.5% by mass based on 100% by mass of the total amount of the compound (B) and the alkali metal (D).

4. The method for producing an olefin dimer according to claim 1, wherein the content ratio of the alkali metal (D) is from 5.2 to 10.5% by mass based on 100% by mass of the total amount of the compound (B) and the alkali metal (D).

5. (Canceled)

6. An olefin dimerization catalyst, comprising: a compound (B) represented by BₙX, wherein B is Na or K, X is CO₃, SO₄, SiO₃, F, Cl, or Br, and n is an integer of 1 or 2 determined by a valence of X; and an alkali metal (D),
wherein the compound (B) has a volume of pores with a pore diameter of from 0.05 µm to 10 µm of from 0.01 mL/g to 0.12 mL/g, and
a content ratio of the alkali metal (D) is from 5.2 to 12.5% by mass based on 100% by mass of the total amount of the compound (B) and the alkali metal (D).

7. The olefin dimerization catalyst according to claim 6, wherein the content ratio of the alkali metal (D) is from 6.2 to 12.5% by mass.

8. The olefin dimerization catalyst according to claim 6, wherein the content ratio of the alkali metal (D) is from 6.2 to 10.5% by mass.

9. The olefin dimerization catalyst according to claim 6, wherein the compound (B) has a volume of pores with a pore diameter of from 0.05 µm to 10 µm of from 0.01 mL/g to 0.09 mL/g.

10. The olefin dimerization catalyst according to claim 6, wherein the compound (B) has a volume of pores with a pore diameter of from 0.05 µm to 10 µm of from 0.01 mL/g to 0.08 mL/g.

11. The olefin dimerization catalyst according to claim 6, comprising a compound (B) represented by BₙX, wherein B is Na or K, X is CO₃, SO₄, SiO₃, F, Cl, or Br, and n is an integer of 1 or 2 determined by a valence of X, and an alkali metal (D),
wherein the compound (B) has a volume of pores with a pore diameter of from 0.05 µm to 10 µm of from 0.01 mL/g to 0.09 mL/g, and
a molar ratio of the molar amount of all alkali metals [T'] to the molar amount of B in the compound (B) [AB'] ([AB']/[T'] (m.r.)) is from 0.60 to 0.89/(m. r.).
